(19) Europäisches Patentamt
European Patent Office
Office européen des brevets

(11) Veröffentlichungsnummer: **0 262 587 B1**

# (12) EUROPÄISCHE PATENTSCHRIFT

(45) Veröffentlichungstag der Patentschrift: **08.01.92**

(51) Int. Cl.[5]: **C07C 233/65**, C07C 231/02, A61K 7/16, A61K 31/165

(21) Anmeldenummer: **87113954.9**

(22) Anmeldetag: **24.09.87**

## (54) Salicylsäureamide, Verfahren zu ihrer Herstellung und ihre Verwendung.

(30) Priorität: **02.10.86 DE 3633501**

(43) Veröffentlichungstag der Anmeldung:
**06.04.88 Patentblatt 88/14**

(45) Bekanntmachung des Hinweises auf die Patenterteilung:
**08.01.92 Patentblatt 92/02**

(84) Benannte Vertragsstaaten:
**AT BE CH DE ES FR GB GR IT LI LU NL SE**

(56) Entgegenhaltungen:

**REVUE DES TRAVAUX CHIMIQUES, vol. 78, 1959, Seiten 207-214, T. KRALT et al.: "Synthesis and pharmacological properties of salicylamides"**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Leinen, Hans Theo, Dr.**
**Gertrudistrasse 2**
**W-4000 Düsseldorf(DE)**
Erfinder: **Lehmann, Rudolf, Dr.**
**Schnugsheide 2**
**W-5653 Leichlingen(DE)**
Erfinder: **Klüppel, Hans-Jürgen, Dr.**
**Begonienstrasse 7**
**W-4000 Düsseldorf(DE)**

**EP 0 262 587 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Salicylsäureamide, ein Verfahren zur Herstellung derartiger Salicylsäureamide sowie die Verwendung der neuen Verbindungen als Wirkstoffe in antimikrobiellen Zusammensetzungen.

Salicylsäure und ihre Derivate sind Verbindungen, von denen seit langer Zeit bekannt ist, daß sie eine physiologische Wirksamkeit aufweisen. Deswegen werden sie in zahlreichen Zusammensetzungen als Analgetika, Spasmolytika, Keratolytika oder Antimikrobika verwendet.

Aus "T. Kralt et al Rev. Trav. Chim. 78, 207 (1959)" bzw. "Beilsteins Handbuch der organischen Chemie" sind N-Alkylamide der Salicylsäure bekannt, in denen am Amido-Stickstoffatom ein Alkylrest mit 4, 6 bzw. 12 C-Atome gebunden ist. Derartige N-Alkyl-salicylsäureamide weisen antipyretische und spasmolytische Eigenschaften auf, für die sogar Struktur-Aktivität-Beziehungen gefunden werden konnten.

Wie in einem der vorliegenden Erfindung zugrunde liegenden umfangreichen Versuchsprogramm gefunden wurde, weisen N-Butyl-salicylsäureamid sowie das entsprechende N-Hexyl- und N-Dodecylhomologe eine äußerst schwache antimikrobielle, d.h. mikrobistatische Wirksamkeit gegenüber zahlreichen Keimen auf. Überraschend wurde jedoch gefunden, daß N-Octyl- und N-Decyl-salicylsäureamid schon bei niedrigen Konzentrationen eine ganz hervorragende Wirkung gegenüber zahlreichen Keimen aufweist. Insbesondere wurde überraschend festgestellt, daß die beiden genannten Verbindungen eine hochselektive Hemmwirkung gegenüber grampositiven Bakterien aufweisen.

Aufgabe der vorliegenden Erfindung war es somit, neue N-Alkylamide der Salicylsäure sowie ein ökonomisches, für die Anwendung im industriellen Bereich geeignetes Verfahren zu ihrer Herstellung zur Verfügung zu stellen. Eine weitere Aufgabe der Erfindung ist darin zu sehen, antimikrobiell wirksame Zusammensetzung zur Verfügung zu stellen, die eine oder beide der oben genannten Verbindungen in möglichst geringen aber antimikrobiell wirksamen Konzentrationen enthalten und die eine selektive Hemmwirkung gegenüber bestimmten Keimen in einer in der Praxis brauchbaren Konzentration zeigen.

Die Erfindung betrifft daher N-Octyl-salicylsäureamid und N-Decyl-salicylsäureamid.

Die Erfindung betrifft weiter ein Verfahren zur Herstellung von N-Octyl-salicylsäureamid und N-decyl-salicylsäureamid aus Salicylsäuremethylester und einem primären Amin, das dadurch gekennzeichnet ist, daß man nach an sich bekannten Methoden Salicylsäuremehthylester mit n-Octylamin und/oder n-Decylamin umsetzt, das gebildete Methanol entfernt und die Reaktionsprodukte in an sich bekannter Weise reinigt.

Die Erfindung betrifft außerdem die Verwendung von N-Octyl-salicylsäureamid und N-Decyl-salicylsäureamid in antimikrobiellen Zusammensetzungen.

N-Octyl-salicylsäureamid und N-decyl-salicylsäureamid sind neue Verbindungen, die aus dem Stand der Technik nicht bekannt sind. Beide Verbindungen weisen überraschenderweise eine hervorragende antimikrobielle Wirkung auf. Lösungen beider Verbindungen oder auch von Mischungen der beiden Salicylsäureamide sind nämlich in der Lage, eine selektive Hemmwirkung gegenüber grampositiven Bakterien zu entfalten. Dies überrascht insofern, als die niederen N-Alkyl-homologen, wie auch die Verbindungen mit längeren Alkylresten am Amido-Stickstoffatom eine derartige Wirkung garnicht oder nur in deutlich geringerem Ausmaß zeigen.

Die erfindungsgemäßen Verbindungen werden nach an sich aus dem Stand der Technik bekannten Verfahren hergestellt. Erfindungsgemäß stellt man N-Octyl-salicylsäureamid und N-Decyl-salicylsäureamid aus Salicylsäuremethylester und dem entsprechendem Amin dadurch her, daß man nach an sich bekannten Methoden Salicylsäuremethylester mit n-Octylamin und/oder n-Decylamin umsetzt, das im Zuge der Amidierungs-Reaktion gebildete Methanol entfernt und die Reaktionsprodukte in an sich bekannter Weise reinigt. Dieser Reaktionsweg wird in dem nachfolgenden Reaktionsschema dargestellt.

Reaktionsschema

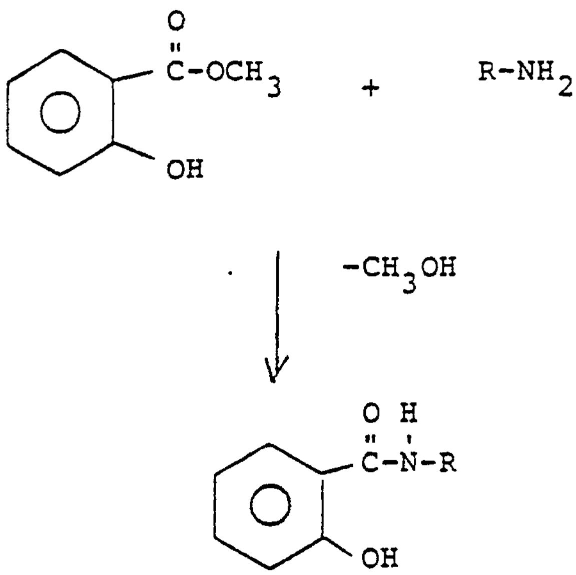

R = n-$C_8H_{17}$, n-$C_{10}H_{21}$

Erfindungsgemäß ist es also möglich, Salicylsäuremethylester mit einem oder beiden primären Aminen umzusetzen. Bei Umsetzung mit einem Amin wird das jeweilige Amid in mehr oder weniger einer Form erhalten, während bei Umsetzung von Salicylsäuremethylester mit einer beliebigen Mischung beider n-Alkylamine die beiden Salicylsäureamide in dem Mengenverhältnis entstehen, in dem die n-Alkylamine in die Reaktion eingesetzt werden. Erfindungsgemäß ist es jedoch bevorzugt, daß zur Amidbildung nur eines der beiden n-Alkylamine eingesetzt wird, um die jeweiligen Salicylsäureamide in mehr oder weniger einer Form zu erhalten.

In einer bevorzugten Ausführungsform des erfindungsgemäßen Herstellungsverfahrens der beiden neuen N-Alkylsalicylsäureamide wird die Reaktion bei erhöhter Temperatur durchgeführt, um sie zu beschleunigen. Die Reaktionstemperaturen liegen üblicherweise zwischen 100 und 200 °C und werden bevorzugt auf einen Bereich von 130 bis 170 °C eingestellt.

Die Reaktion der Bildung der Salicylsäureamide gemäß der Erfindung kann in einem Lösungsmittel durchgeführt werden. Als Lösungsmittel werden - wie aus dem Stand der Technik an sich bekannt - aprotische organische Lösungsmittel, wie beispielsweise Diethylether verwendet. In einer bevorzugten Ausführungsform der erfindungsgemäßen Reaktion ist es jedoch möglich, die Amidierungsreaktion in Abwesenheit eines Lösungsmittels durchzuführen, da beide Ausgangsstoffe flüssig sind und sich ohne weiteres unter den angegebenen Reaktionsbedingungen miteinander zur Reaktion bringen lassen. In diesem Fall wird das Entfernen des Lösungsmittels, beispielsweise durch Abdestillieren, als Arbeitsgang gespart. Die Durchführung der Reaktion in Abwesenheit eines Lösungsmittels ist also als bevorzugte Ausführungsform anzusehen.

In einem der eigentlichen Amidbildung nachgelagerten Reaktionsschritt wird das im Verlauf der Reaktion gebildete Methanol aus der Reaktionsmischung entfernt. Dies geschieht ebenfalls nach aus dem Stande der Technik bekannten Methoden. Bevorzugt wird das Reaktionsprodukt Methanol abdestilliert und dabei - zur Entfernung auch letzter Methanolreste - Vakuum angelegt und die Temperatur auf über 100 °C, bevorzugt über 130 °C, angehoben. In vorteilhafter Weise kann man - bei Abdestillation des Methanols gleich nach Abschluß der Amidierungsreaktion - die noch verbliebene Wärme der Reaktionsmischung für die Einstellung eines höchsten Temperaturwertes bei der Abdestillation des Methanols nutzen, ohne daß erneut Energie in die Anhebung der Reaktionsmischung aufgewendet werden muß. Dies geht in bevorzugter Weise dann, wenn - wie oben als bevorzugt beschrieben - in Abwesenheit eines Lösungsmittels gearbeitet wurde, da die Reaktionswärme dann nicht schon für das Abdestillieren des Lösungsmittels benötigt wurde.

Die entstandenen Reaktionsprodukte werden dann auf ebenfalls an sich bekannte Weise gereinigt. Dies geschieht in vorteilhafter Weise dadurch, daß man eventuell nicht umgesetztes Amin, das in die Reaktion üblicherweise in äquimolaren Mengen mit dem Salicylsäu remethylester eingesetzt wird, durch Auswaschen mit wäßriger Säure und anschließendes Behandeln der Reaktionsmischung mit Natriumhydrogencarbonat-Lösung entfernt, und die so erhaltenen Kristalle der Reaktionsprodukte durch Umkristallisieren in einem

geeigneten Lösungsmittel bzw. Lösungsmittelgemisch reinigt. Als Lösungsmittel werden üblicherweise unpolare Kohlenwasserstoffe oder aprotische organische Lösungsmittel verwendet; aus der großen Klasse dieser Lösungsmittel sind n-Alkane wie n-Hexan oder Ether wie Diethylether besonders gut geeignet. Es ist jedoch auch möglich, Lösungsmittelgemische zur Umkristallisation zu verwenden; als solche haben sich beispielsweise Mischungen von Methanol und Wasser bewährt.

Die gewünschten N-Octyl- und N-Decyl-salicylsäureamide entstehen bei dem erfindungsgemäßen Verfahren in hohen Ausbeuten: so ist es möglich, Salicylsäuredecylamid nach dem Umkristallisieren in einer Gesamtausbeute von 75 % der Theorie in reiner Form zu erhalten.

Als Ausgangsstoff wird in das erfindungsgemäße Verfahren üblicherweise Salicylsäuremethylester aus nativen Quellen eingesetzt: sogenanntes "Wintergrünöl", das zu über 99 % aus Salicylsäuremethylester besteht, ist als Edukt für die Amidierungsreaktion in hervorragender Weise geeignet. Es ist jedoch auch möglich, Salicylsäuremethylester synthetischen Ursprungs zu verwenden.

Wie schon weiter oben beschrieben, zeigen N-Octyl-salichlsäureamid und N-Decyl-salicylsäureamid schon in geringen Konzentrationen, beispielsweise bei 2,5 bis 10 ppm des jeweiligen Amids in der Anwendungslösung, eine hervorragende und selektive Hemmwirkung gegenüber bestimmten Keimen. Dies ist insofern überraschend, als sowohl die höheren Homologen, wie N-Dodecyl- und N-Tetradecyl-salicylsäureamid, als auch die niederen Homologen, wie N-Butyl- und N-Hexyl-salicylsäureamid, eine nur wesentlich schwächere Hemmwirkung gegenüber den selben Keimen zeigen. Die erfindungsgemäßen Verbindungen eignen sich daher hervorragend zur Verwendung in antimikrobiellen Zusammensetzungen, die nicht auf mikrobicide Eigenschaften der Wirkstoffe abzielen, sondern eher auf die mikrobistatischen Eigenschaften. Solche antimikrobiellen Zusammensetzungen, die N-Octyl-salicylsäureamid und/oder N-Decyl-salicylsäureamid enthalten, sind in der Regal wäßrige bzw. Wasser-kompatible Systeme und können auch noch weitere Bestandteile enthalten, die in derartigen antimikrobiellen Zusammensetzungen üblicherweise enthalten sind. Neben dem Hauptbestandteil Wasser können dies beispielsweise Duftstoffe, Tenside oder andere, an sich bekannte Verbindungen sein, die mit den beiden erfindungsgemäßen Amiden kompatibel sind und diese in ihrer Wirkung nicht beeinträchtigen.

Bevorzugt ist die Verwendung der Verbindungen gemäß der vorliegenden Erfindung in Zusammensetzungen, die gegen grampositive Keime eingesetzt werden sollen. Wie sich nämlich zeigte, besitzten die beiden Verbindungen eine herausragende selektive Hemmwirkung gegenüber grampositiven Bakterien, wie beispielsweise Staphylococcus aureus, Streptococcus mutans oder Actinomyces viscosus. Dabei sind bereits sehr geringe Konzentrationen eines oder beider Amine ausreichend (2,5 bis 10 ppm in der Anwendungslösung), um eine gute Hemmwirkung gegenüber diesen grampositiven Keimen zu erzielen.

Aufgrund ihrer selektiven Wirkung gegenüber grampositiven Bakterien können die erfindungsgemäßen Verbindungen alleine oder in Kombination miteinander überall dort mit Vorteil eingesetzt werden, wo es darum geht, grampositive Bakterien gezielt zu bekämpfen. Dies ist beispielsweise der Fall auf dem Gebiet der Mund- und Zahnpflegemittel. Hier ist bekannt, daß grampositive Bakterien bei der Bildung des Zahnbelags und der dadurch ausgelösten Kariesentwicklung eine besondere Rolle spielen. Die erfindungsgemäßen Verbindungen können hier als "Antiplaque-Wirkstoffe" in Mitteln für die Mund- und Zahnpflege, beispielsweise in Mundwässern und Zahnpasten, eingesetzt werden.

Ein weiterer Anwendungsbereich für die erfindungsgemäßen Verbindungen liegt auf dem Gebiet der Körperdeodorantien. Hier ist es bekannt, daß auf der Haut angesiedelte geruchsbildende Bakterien vorwiegend aus der grampositiven Gruppe stammen. Die erfindungsgemäßen Verbindungen können hier als Deo-Wirkstoffe eingesetzt werden.

Weiterhin ist es möglich, die erfindungsgemäßen Verbindungen in Wundbehandlungsmitteln zu verwenden, um bei der Wundbehandlung ein Überwuchern grampositiver Keime, beispielsweise der Bakterien der obengenannten Art, zu verhindern.

Ein weiteres wichtiges Anwendungsgebiet für die erfindungsgemäßen Verbindungen ist die Herstellung von antimikrobiell wirksamen Kombinationspräparaten, die eine möglichst breites und ausgeglichenes Wirkungsspektrum gegenüber den in der Praxis anzutreffenden Keimen aufweisen sollen. Hier können die erfindungsgemäßen Verbindungen in Kombination mit anderen antimikrobiellen Wirkstoffen eingesetzt werden, um sicherzustellen, daß solche Präparate keine Wirkungslücken gegenüber grampositiven Keimen besitzen.

Die Erfindung wird durch die nachfolgenden Beispiele näher erläutert.

Beispiel 1

Darstellung von Salicylsäuredecylamid

1 Mol (152,15 g) Salicylsäuremethylester und 1 Mol (157,30 g) Decylamin wurden ca. 18 Stunden lang unter Rückfluß bei 160 °C Badtemperatur erhitzt. Anschließend wurde das gebildete Methanol aus der Reaktionsmischung abdestilliert und Methanolreste im Vakuum (1,3 mbar/140 °C) entfernt. Die Rohausbeute betrug 265 g.

Zur Reinigung wurde das Rohprodukt in 600 ml Diethylether gelöst und die klare etherische Lösung zweimal mit je 200 ml 5 %iger wäßriger Salzsäure und anschließend mit Natriumhydrogencarbonat gewaschen; letztlich folgte ein weiterer Waschvorgang mit Wasser. Die etherische Phase wurde über Natriumsulfat getrocknet, filtriert und der Diethylether abkondensiert. Die erhaltene Kristallmasse wurde aus n-Hexan umkristallisiert; gleicherweise ist eine Umkristallisation aus einer Methanol/Wasser-Mischung (Volumenverhältnis 1 : 1 möglich. Die Ausbeute an gereinigtem N-Decylsalicylsäureamid betrug 209 g; dies entspricht einer theoretischen Ausbeute von 75 %, bezogen auf die eingesetzten Ausgangsstoffe.

Das Produkt wies folgende physikalische Parameter auf:

Fp. 59 bis 60 °C;

$^1$H-NMR (CDCl$_3$): $\delta$ = 0,87 - 1,68 (m, 19H, $CH_3-(CH_2)_8-$);

3,43 (q, 2H, $(CH_2)_8-\underline{CH_2}-N(H)-$);

12,5 ppm (m, 1H, OH) ppm.

IR (KBr): 3410 cm$^{-1}$ (OH); 3060 cm$^{-1}$ (C-H);

1645, 1590 cm$^{-1}$ (Amid I + II)

Elementaranalyse

$C_{17}H_{27}NO_2$ (277, 41)

Ber.: C 73,61 H 9,81 N 5,05

Gef.: C 73,40 H 10,00 N 4,81

Beispiel 2

Darstellung von N-Octyl-salicylsäureamid

In der in Beispiel 1 beschriebenen Weise wurde aus Salicylsäuremethylester und n-Octylamin (Einsatzmengen 1 Mol : 1 Mol) N-Octyl-salicylsäureamid hergestellt und in gleicher Weise, wie dies in Beispiel 1 beschrieben wurde, gereinigt und umkristallisiert.

Das erhaltene Produkt wies folgende Parameter auf:

$^1$H-NMR (CDCl$_3$): $\delta$ = 0,86 - 1,76 (m, 15 H, $CH_3(CH_2)_6$);

3,40 (9,2 H, $-\underline{CH_2}-NH-$);

6,63 - 7,50 (m, 5H, 4 arom. H, N-H);12,49 (s, 1H, OH)

IR (Schmelze): 3410 cm$^{-1}$ (OH); 3060 cm$^{-1}$ (CH)

1645, 1590 cm$^{-1}$ (Amid I + II)

Fp: 44 - 46 °C (umkristallisiert aus n-Pentan)

Elementaranalyse

$C_{15}H_{25}NO_2$ (249,36)

Ber.: C 72,25 H 9,30 N 5,62

Gef.: C 71,80 H 9,42 N 5,53

Vergleichsbeispiel 1

In gleicher Weise wie in Beispiel 1 beschrieben, wurden die aus dem Stand der Technik bekannten N-Butyl-, N-Hexyl-, N-Dodecyl- und N-Tetradecyl-salicylsäureamide hergestellt und gereinigt. Die Schmelzpunkte der kristallin erhalten Verbindungen sind der nachfolgenden Tabelle 1 zu entnehmen.

Tabelle 1

Schmelzpunkte der Salicylsäureamide

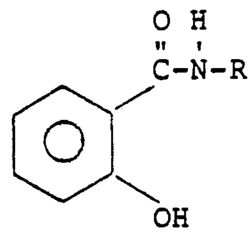

| R ≡ | Fp |
|---|---|
| $C_4H_9$ | (hochviskoses Öl (im Kugelrohr destilliert)) |
| $C_6H_{13}$ | 36-37 (Lit.: 42-43) |
| $C_{12}H_{25}$ | 64-70 (Lit. 70-77°C) |
| $C_{14}H_{29}$ | 74-77 |

Beispiel 3

Die mikrobistatische Wirksamkeit der erfindungsgemäßen Salicylsäureamide sowie ihrer aus dem Stand der Technik bekannten niederen bzw. höheren Homologen (des Vergleichsbeispiels 1) wurde gegenüber folgenden Testkeimsuspensionen bestimmt:

1. Staphylococcus aureus: $2 \times 10^9$ Keime/ml
2. Streptococcus mutans: $1 \times 10^9$ Keime/ml
3. Actinomyces viscosus: $2 \times 10^8$ Keime/ml.

Die Hemmkonzentrationen der zu untersuchenden Verbindungen wurden mit Hilfe des Verdünnungstestes nach den Richtlinien für die Prüfung und Bewertung chemischer Desinfektionsverfahren der Deutschen Gesellschaft für Hygiene und Mikrobiologie, abgedruckt in Zbl. Bakt. Hyg. I. Abt. Orig. B 172, 536-537 (1981), ermittelt. Die Versuche wurden in sterilen Reagenzröhrchen ausgeführt, die Standard-I-Bouillon (pH 7,5 Merck) oder Brain-heart-Medium (pH 7,4; Difco, USA) enthielten. Nach Zugabe der Wirkstoffe betrug das Nährlösungsvolumen in den Röhrchen jeweils 5 ml. Anschließend wurden jeweils 0,1 ml der Testkeimsuspension der angegebenen Konzentration in die Röhrchen gebracht. Die mit Bakterien beimpften Nährlösungsproben wurden 3 Tage lang bei 37°C im Brutschrank aufbewahrt. Die mit Actinomyces viscosus beimpften Proben wurden bei 30°C unter anaeroben Bedingungen aufbewahrt. Danach wurde festgestellt, welche dem Nährmedium zugeführte Wirkstoffkonzentration das Wachstum der Keime gerade noch gehemmt hatte. Der auf diese Weise gefundene Wert wurde als Hemmkonzentration bezeichnet. Folgende Wirkstoffkonzentrationen (ppm) wurden getestet: 1000, 500, 250, 100, 50, 10, 5 und 2,5.

Die Angaben für die Hemmkonzentration sind der nachfolgenden Tabelle 2 zu entnehmen.

Vergleichsbeispiel 2

In gleicher Weise wie in Beispiel 3 beschrieben, wurde die mikrobistatische Wirksamkeit der Verbindungen des Vergleichsbeispiels 1 gegenüber den genannten Testkeimen bestimmt. Die Ergebnisse sind ebenfalls der nachfolgenden Tabelle 2 zu entnehmen.

Tabelle 2

Mikrobistatische Wirkung der Verbindung ermittelt im Röhrchentest 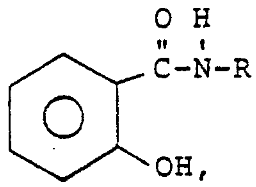

| Verb. aus Beisp. | R = | Hemmkonzentration (ppm) gegenüber | | |
|---|---|---|---|---|
| | | Staph. aureus | Strept. mutans | Actinomyces viscosus |
| 1 | $n\text{-}C_{10}H_{21}$ | 5 | 5 | 2.5 |
| 2 | $n\text{-}C_{8}H_{17}$ | 5 | 5 | 10 |
| Vgl.-Bsp.1 | $n\text{-}C_{4}H_{9}$ | 250 | 500 | 500 |
| " | $n\text{-}C_{6}H_{13}$ | 50 | 50 | 50 |
| " | $n\text{-}C_{12}H_{25}$ | 500 | n.g.[1)] | n.g.[1)] |
| " | $n\text{-}C_{14}H_{29}$ | 250 | n.g.[1)] | n.g.[1)] |

1) n.g. = nicht geprüft.

Ergebnis:

Die erfindungsgemäßen Verbindungen zeigen gegenüber den genannten Testkeimen eine deutlich bessere Wirkung als ihre niederen bzw. höheren Homologen: die Hemmkonzentrationen lagen zum Teil um den Faktor 100 unter denen der Vergleichsverbindungen.

## Patentansprüche

1. N-Octyl-salicylsäureamid und N-Decyl-salicylsäureamid.

2. Verfahren zur Herstellung von N-Octyl-salicylsäureamid und N-Decyl-salicylsäureamid aus Salicylsäuremethylester und einem primären Amin, dadurch gekennzeichnet, daß man nach an sich bekannten Methoden
   a) Salicylsäuremethylester mit n-Octylamin und/oder n-Decylamin umsetzt,
   b) das gebildete Methanol entfernt und
   c) die Reaktionsprodukte in an sich bekannter Weise reinigt.

3. Verfahren nach Anspruch 2, dadurch gekennzeichnet, daß man zur Amidbildung nur ein Amin einsetzt.

4. Verfahren nach Ansprüchen 2 und 3, dadurch gekennzeichnet, daß man Salicylsäuremethylester und

ein Amin bzw. beide Amine im Molverhältnis 1 : 1 einsetzt.

5. Verfahren nach Ansprüchen 2 bis 4, dadurch gekennzeichnet, daß man die Reaktion der Amidbilding bei erhöhter Temperatur durchführt.

6. Verfahren nach Ansprüchen 2 bis 5, dadurch gekennzeichnet, daß man die Reaktion in Abwesenheit eines Lösungsmittels durchführt.

7. Verfahren nach Ansprüchen 2 bis 6, dadurch gekennzeichnet, daß man das gebildete Methanol im Vakuum abdestilliert.

8. Antimikrobielles Mittel für nichttherapeutische Zwecke, dadurch gekennzeichnet, daß N-Octylsalicylsäureamid und/oder N-Decylsalicylsäureamid enthalten ist.

9. Mundwasser, dadurch gekennzeichnet, daß N-Octylsalicylsäureamid und/oder N-Decylsalicylsäureamid enthalten ist.

10. Zahnpasta, dadurch gekennzeichnet, daß N-Octylsalicylsäureamid und/oder N-Decylsalicylsäureamid enthalten ist.

11. Körperdeodorant, dadurch gekennzeichnet, daß N-Octylsalicylsäureamid und/oder N-Decylsalicylsäureamid enthalten ist.

**Claims**

1. N-octyl salicylic acid amide and N-decyl salicylic acid amide.

2. A process for the production of N-octyl salicylic acid amide and N-decyl salicylic acid amide from salicylic acid methyl ester and a primary amine, characterized in that

   a) salicylic acid methyl ester is reacted with n-octylamine and/or n-decylamine by methods known per se,
   b) the methanol formed is removed and
   c) the reaction products are purified by methods known per se.

3. A process as claimed in claim 2, characterized in that only one amine is used for the amidation.

4. A process as claimed in claims 2 and 3, characterized in that salicylic acid methyl ester and one amine or both amines are used in a molar ratio of 1 : 1.

5. A process as claimed in claims 2 to 4, characterized in that the amidation reaction is carried out at elevated temperature.

6. A process as claimed in claims 2 to 5, characterized in that the reaction is carried out in the absence of a solvent.

7. A process as claimed in claims 2 to 6, characterized in that the methanol formed is distilled of in vacuo.

8. An antimicrobial agent for non-therapeutic purposes, characterized in that it contains N-octyl salicylic acid amide and/or N-decyl salicylic acid amide.

9. A mouthwash, characterized in that it contains N-octyl salicylic acid amide and/or N-decyl salicylic acid amide.

10. A toothpaste, characterized in that it contains N-octyl salicylic acid amide and/or N-decyl salicylic acid amide.

11. A personal deodorant, characterized in that it contains N-octyl salicylic acid amide and/or N-decyl

salicylic acid amide.

**Revendications**

1. N-octyl salicylamide et N-décyl salicylamide.

2. Procédé d'obtention de la N-octyl salicylamide et de la N-décyl salicylamide à partir de salicylate de méthyle et d'une amine primaire, caractérisé en ce que :
   a) selon les méthodes connues, on met en réaction le salicylate de méthyle avec la n-octylamine et/ou la n-décylamine,
   b) on élimine le méthanol formé et
   c) on purifie les produits de la réaction d'une manière connue en soi.

3. Procédé selon la revendication 2, caractérisé en ce que l'on met en oeuvre seulement une amine en vue de la formation d'amide.

4. Procédé selon les revendications 2 et 3, caractérisé en ce que l'on met en oeuvre le salicylate de méthyle et une ou les deux amines dans un rapport molaire 1:1.

5. Procédé selon les revendications 2 à 4, caractérisé en ce que la réaction d'amidification est effectuée à température élevée.

6. Procédé selon les revendications 2 à 5, caractérisé en ce que l'on effectue la réaction en l'absence d'un solvant.

7. Procédé selon les revendications 2 à 6, caractérisé en ce que le méthanol formé est séparé par distillation sous vide.

8. Composition anti-microbienne pour des fins non thérapeutiques, caractérisée en ce qu'elle contient la N-octyl salicylamide et/ou la N-décyl salicylamide.

9. Bain de bouche caractérisé en ce qu'ils contiennent de la N-octyl salicylamide et/ou de la N-décyl salicylamide.

10. Pâtes dentifrices caractérisées en ce qu'elles contiennent de la N-octyl salicylamide et/ou de la N-décyl salicylamide.

11. Désodorisant corporel caractérisé en ce qu'il contient de la N-octyl salicylamide et/ou la N-décyl salicylamide.